# EUROPEAN PATENT SPECIFICATION

(11) **EP 0 469 515 B1**
(45) Date of publication and mention of the grant of the patent: **30.11.1994**
(21) Application number: 91112710.8
(22) Date of filing: 29.07.1991
(51) Int. Cl.: A61J 1/00

(54) **Container for preserving blood for testing**
Behälter zum Aufbewahren von Blut zur Untersuchung
Récipient pour conserver le sang afin de réaliser des tests

(30) Priority: 02.08.1990 JP 82296/90; 13.06.1991 JP 168670/91
(43) Date of publication of application: 05.02.1992
(73) Proprietor: KAWASUMI LABORATORIES, INC., Tokyo (JP)
(72) Inventor: Juji, Takeo c/o the Medical Department,, Bunkyo-ku, Tokyo (JP); Takahashi, Koki c/o the Medical Department, Bunkyo-ku, Tokyo (JP); Kagawa, Yoichi c/o Kawasumi Laboratories, Inc., Tokyo (JP)
(74) Representative: Heusler, Wolfgang, Dipl.-Ing.

(56) References cited:
- EP-A- 0 054 221
- EP-A- 0 074 178
- EP-A- 0 114 964
- EP-A- 0 186 018

## Description

### Background of the Invention

### Field of the Invention

The invention relates to a container to preserve blood for testing which enables to achieve various blood tests at high accuracy even after preserving collected blood for a long time.

### Description of the Prior Art

In organ transplantation or marrow transplantation, particularly in the latter, it is necessary to confirm that the histocompatible antigens or a donor and a recipient be compatible before transplantation. HLA, Human Leukocyte Autigen, is the major histocompatible antigen system in man.

First, after collecting lymphocytes of a donor and a recipient, the type of HLA shall be classified by having them react with various anti-HLA antisera.

Second, a compartible donor is selected by comparing HLA of the donors and the recipients.

Finally, their histocompatibility is confirmed by examining MLC (Mixed Lymphocyte Culture) reaction.

Only 20 to 30% of the patients in need of marrow transplantation can find an HLA histocompatible related donor from their blood relatives. Other patients have to rely on the donation of marrow by a histocompatible unrelated donors. Because of this, an organization (marrow bank) which requests the cooperation of a third party in good faith, carries out typing and registering the type of HLA of many marrow donor candidates and provides histocompatible marrow to patients who need unrelated bone marrow transplantion is established.

As a conventional container to preserve blood test samples, a vacuum blood collecting tube made of glass which inside is made vacuum has been used. However, there is such a time restriction in this vacuum blood collecting tube that the above described HLA typing test has to be carried out within 24 hours after collecting blood unless a special operation such as separating lymphocytes quickly after collecting blood and freeze-preserving the lymphocytes is performed.

From the document EP-A-0054221 is known a multiple bag system for the collection, storage or processing of whole blood into various blood components under sterile conditions, which comprises at least two bags inter-connected by a flexible conduit. For enabling the storage of the collected blood up to about 21 days at the standard blood storage temperature of about 5°C, the bags of this known system are made of a material showing good carbon dioxyde and oxygen transmissibility.
Further, from the EP-A-0074178 is known a container made of a copolymeric film for platelet storage. The film material from which the storage container is made has special permeabilities for oxygen and carbon dioxyde for maintaining the viability of the platelet for 5 or 7 days.
Finally, from the EP-A-0186018 is known a storage bag for storing whole blood, erythrocyte or thrombocyte concentrate, which has a gas permeability of at least 800 cm³ O₂ and at least 4000 cm³ CO₂ per day/m²/bar, for allowing storage of the blood components for a duration of at least four weeks at 4 to 6°C.

### Summary of the invention

It is an object of the present invention to provide a container for preserving blood to be supplied to HLA histocompatible test, which enables to preserve blood samples in good condition for testing for at least three days at room temperature, while maintaining the viability of the lymphocytes not less than 90%. Another object of the invention is to provide a container for collected blood by aseptic operation and preserving blood components for a long period of time keeping aseptic.
According to the present invention, there is provided a container for preserving blood to be supplied to HLA histocompatible test, in which lymphocytes contained in the blood are kept for at least three days at room temperature, while maintaining the viability of the lymphocytes not less than 90 %, characterized by providing a main body container composed of a flexible synthetic resin having oxygen permeability of not less than 0.1 ml/24 h·atm per 1 ml of a whole blood, and having a capacity of 20 to 80 ml, a sampling port portion provided in said main body container at an upper end thereof, having a rubber made button for piercing a needle, and an anti-coagulant agent enclosed in advance in said main body container.
Preferably, the inventive container for preserving the blood to be tested is characterized by providing a blood collecting means furnished with under mentioned structure in the main body container, a tube having a pricking needle at its end portion for introducing the blood from a donor into the container through the needle, a connecting member interposed on the way of said blood introducing tube, having a closing member for closing the flow path of said tube for introducing the blood when not used and having a structure that the flow path is opened by breaking the closing member when used, a sampling port portion interposed on the way of said blood introducing tube, having a rubber made button for piercing a needle, and a clamp provided between the sampling port portion of the blood introducing tube and the connecting member.
According to a special embodiment, this inventive container is characterized by a first connecting tube connected to the main body container via a connecting member, having branched tubes, a first container for holding blood components, connected to one branched tube of said first connecting tube and having a sampling port portion on the upper end thereof, a second container for holding the blood components, connected to the other branched tube of said first connecting tube via a connecting member, a container for enclosing a diluting liquid, connected to said second container via the second connecting tube, a sampling port portion provided in said second container and a connecting mouth of the second connecting tube, and a connecting member provided in said diluting liquid container and the connecting mouth of the second connecting tube.
According to the invention it can be provided that the container for preserving the blood to be tested is characterized in that said connecting members are integrally formed with a large diametered tubular piece of synthetic resin and a small diametered piece for closing the upper end of said large diametered piece, and when the small diametered piece is broken, the upper end of the large diametered piece is opened, so that the paths of said blood introducing tubes are opened.

### Brief Description of the Drawings

Fig. 1 is a schematic drawing of the container to preserve blood for testing according to the invention.

Fig. 2 is a schematic drawing showing another example of the container to preserve blood for testing according to the invention.

Fig. 3 is a schematic drawing showing still another example of the container for preserving blood for testing according to the invention.

Fig. 4 is a graph showing the relation between days of preservation and the viability of lymphocytes when the lymphocytes are preserved using the container for preserving blood for testing according to the invention.

Fig. 5 is a graph showing the relation between the days of preservation and stimulation index when lymphocytes of blood of donor and patient are mixedly cultured using a container for preserving blood for testing according to the invention to examine the reactivity of T lymphocytes.

### Description of the Preferred Embodiments

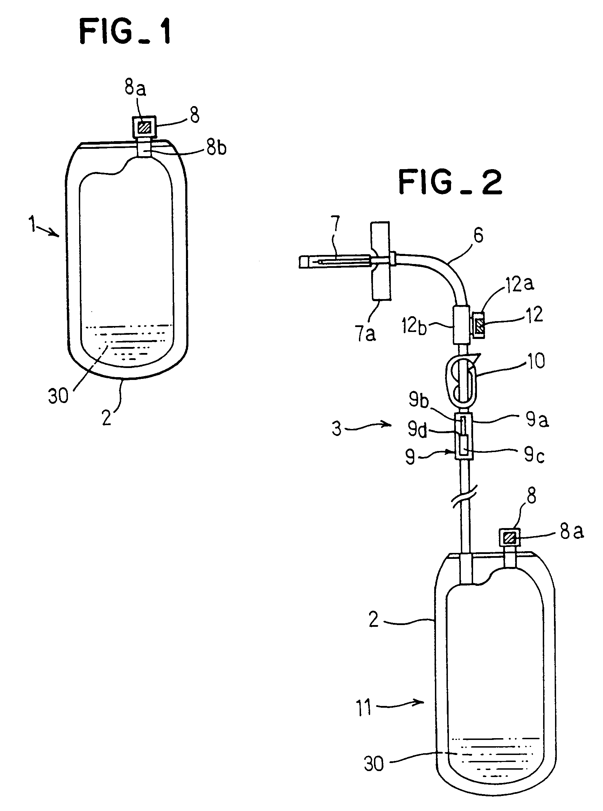

Fig. 1 is a schematic drawing of the container to preserve blood for test according to the invention. A container 1 for preserving is provided with a sampling port part 8 in the upper end part of a bag-like main body container 2 made of flexible synthetic resin.

As flexible synthetic resin forming the above main body container 2, a material having oxygen permeability of not less than 0.10 ml/24 h/atm per 1 ml of whole blood is used. For example, synthetic resin such as soft vinyl chloride resin, soft vinyl chloride based elastomer (for example, Esmedica, a commodity of Sekisui Kagaku), styrene based elastomer such as poly (ethylenebutylene) polystyrene block copolymer (for example, Craytone G, a commodity of Shell Chemical; Toughteck, a commodity of Asahi Kasei Kogyo), polypropylene, bridged products of ethylene-vinyl acetate copolymer (EVA), olefine based elastomer (for example, Dominos, a commodity of Nihon Polyurethane), polyethyleneterephthalate or copolymers thereof and silicone resin. The oxygen permeability is restricted within the range described above in order to keep the taking in of oxygen and carbon dioxide and the release thereof by metabolism of blood component at a level not lower than a proper level.

The main body container 2 is constructed by piling two sheets made of the above flexible synthetic resin, heat-sealing the circumference of the above sheets with the state that sampling port part 8 being inserted to the upper end part thereof and making them bag-like.

The sampling port part 8 is provided so as to be able to sample blood for testing contained in the main body container 2 through a pricking needle or inject blood for testing. As the sampling port part 8, a so-called mixed injecting part, which is used in a blood circulation circuit and the like, is used. That is, a button made of rubber is set to the widened port part of the end part of a tube 8b, and the button made of rubber is covered with a cap. This cap is provided with an opening part so that the button made of rubber can be pricked with a needle. Since there are cases that the button made of rubber is pricked with a needle a plurality of times in the invention, it is preferred that the button made of rubber is press-set to the above widened port part of the tube and to the cap using an ultrasonic welder so that a hole formed on the button by being pricked with a needle can easily be closed after pricking.

In order to improve the preservability of blood and blood components, an anti-coagulating agent 30 such as an ACD liquid (containing citric acid and glucose as main ingredients), a CPD liquid (containing citric acid and glucose as main ingredients), or a 4% aqueous solution of sodium citrate or the like is enclosed inside the above main body container 2.

It is preferred that the volume of the above main body container 2 is 20 to 80 ml. The reason for this is that if it is not larger than 20 ml, it is not possible to keep blood for testing at a sufficient amount, and if it is not less than 80 ml, blood is collected at a larger amount than required, so that excess blood is wasted.

Fig. 2 shows another example of the invention. In a container 11 for preserving, a blood collecting tool 3 is attached to the upper end of a main body container 2. The blood collecting tool 3 has a soft tube 6 for introducing blood, a pricking needle 7 attached to the end part of the tube 6, a connecting member 9 placed in the middle of the tube 6, a sampling port part 12 placed in the tube 6 on its end side than the connecting member 9 and a clamp 10 placed in the middle between the connecting member 9 and the sampling port part 12.

The pricking needle 7 is designed so that after a blood vessel of a blood donor is pricked with the pricking needle, a wing 7a is fixed to his arm or the like by means of an adhesive tape.
The connecting member 9 is provided with a soft tube 9a which upper and lower ends are connected to the above tube 6 for introducing blood, a large diameter piece 9c having a cylindrical shape made of hard resin inserted into the soft tube 9a, and a small diameter piece 9b molded integrally with said large diameter piece 9a closing the upper end part of the large diameter piece 9c. When the small diameter piece 9b is pressed from the exterior of the soft tube 9a with fingers or the like, a thin part 9d formed in the boundary part between the large diameter piece 9c and the small diameter piece 9d is broken and the upper end part of the large diameter piece 9c is opened, whereby a liquid flows through the large diameter piece 9c into the tube 6 for introducing blood.

The sampling port part 12 is provided in a large diameter tube 12b connected to the tube 6 for introducing blood and has a button 12a made of rubber for mixed injection. The structure of this button 12a made of rubber for mixed injection in the sampling port part 12 is the same as that of the above described sampling port part 8. The structure of the main body container 2 is the same as that of the container 1 illustrated in Fig. 1 so that its explanation is omitted.

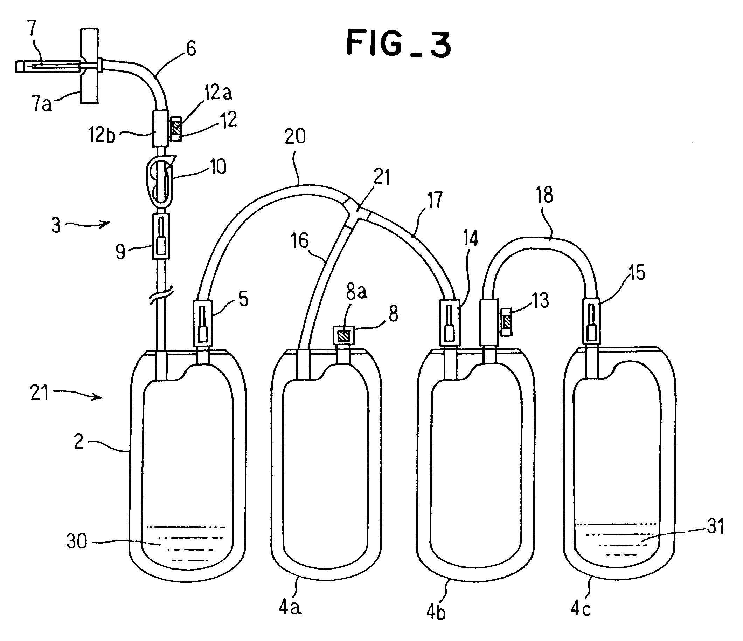

Fig. 3 shows still another example of the invention. A main body container 21 for preserving has a main body container 2 to which the above described blood collecting tool 3 is connected, containers 4a and 4b for separating blood components and a container 4c for enclosing a diluting liquid. Instead of the sampling port part 8 in the above container 11 for preserving, a connecting member 5 is connected to the upper end part of the main body container 2, and a connecting tube 20 is connected to the connecting member 5. A branching tube 21 is connected to the connecting tube 20, and connecting tubes 16 and 17 are connected to the branching tube 21. The end of the connecting tube 16 is connected to the upper end part of the container 4a for separating blood components, and the end part of the connecting tube 17 is connected through a connecting member 14 to the container 4b for separating blood components.

To the upper end part of the container 4a for separating blood components, a sampling port part 8 having a button 8a made or rubber for mixed injection is attached. Furthermore, the container 4b for separating blood components and the container 4c for enclosing diluting liquid are connected with each other through a connecting tube 18, and a sampling port part 13 is attached to the connecting tube 18 at the upper end part of the container 4b for separating blood components. A diluting liquid 31 such as a physiological salt aqueous solution, a phosphoric acid buffer solution or the like is enclosed in the container 4c for enclosing a diluting liquid.

The material for the containers 4a, 4b and 4c, the structure of the connecting member 5, 14 and 15, and the structure of the sampling port part 13 are the same as those explained in Fig. 1 or Fig. 2. The connecting tubes 16, 17, 18 and 20 are made of soft synthetic resin.

Examples of the application of the container 21 for preserving shown in Fig. 3 are explained. Firstly, after pricking a blood vessel of a blood donor with the prick needle 7, blood is introduced to the main body container 2 through the tube 6 for introducing blood and preserved. Before carrying out a blood test, this preserved blood in the main body container 2 by subjecting centrifuge separation of the container 21 for preserving segregate into blood components of a plasma layer/ a buffy coat layer/ an erythrocyte layer. Thereafter, the connecting member 5 of the main body container 2 is opened, and the main body container 2 is gradually pressed from the bottom to the top to thereby extrude the supernatant plasma layer and transfer it through the connecting tubes 20 and 16 to the container 4a for separating blood components. Then, after closing the connecting tube 16 with a clamp or the like, which is not shown in the figure, or cutting the connecting tube 16 by heat-sealing, the connecting means 14 of the container 4b for separating blood components is opened, and the buffy coat layer in the main body container 2 is transferred in the same manner as described above through the connecting tubes 20 and 17 to the container 4b for separating blood components. After finishing these operations, the erythrocyte layer remains in the main body container 2.

The blood components in the above main body containers 2, 4a and 4b are collected by pricking the sampling port parts 12, 8 and 13 with a blood collecting needle of a syringe or the like. The erythrocyte layer in the main body container 2 is used for test of erythrocytes, the plasma layer in the container 4a for separating is used for test of plasma, proteins, platelets and the like and the buffy coat layer in the container 4b for separating is used for test of lymphocytes. There are cases that the lymphocytes in the buffy coat layer do not have a sufficient amount of liquid. In such cases, the lymphocytes can be diluted by opening the connecting part 15 of the container 4c for enclosing a diluting liquid and introducing the diluting liquid 31 through the connecting tube 18 to the container 4b for separating.

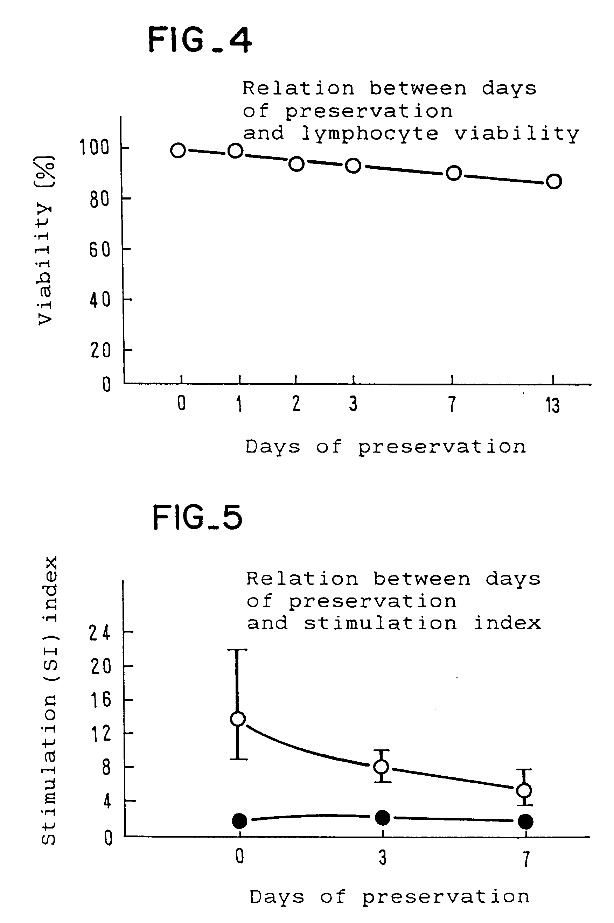

Fig. 4 shows the relation between the days of preservation of preserved lymphocytes collected from the main body container 2 and the viability of the lymphocytes. From this figure, it is understood that the activity of the preserved lymphocytes is kept at not less than 95% the initial value even at 13th day of preservation.

Next, lymphocytes of blood-donors A and B collected and preserved using the above container 21 for preserving were mixedly cultured to examine the reactivity of T lymphocytes (responder). Firstly, the lymphocytes of the blood donor A or B were treated as stimulating cells (Stimulater) by irradiating them with X ray (or MMC) to eliminate their ability of propagation. The above lymphocytes were mixed with those to react therewith, that is, T lymphocytes of the donor A or B, and the reactivity of T lymphocytes was examined by stimulating and measuring the degree of cellular propagation based on the incorporation of 3H-thymidine.

Fig. 5 shows the results of the examination, wherein black dots indicate the stimulation index between the same blood donor A-A or B-B, and an white circles indicate the stimulation index between different blood donors A-B. In this figure, the stimulation indexes of the preserved lymphocytes show a ratio that permits to judge the culture of mixed lymphocytes even after 3 days of preservation. Actually, the culture of mixed lymphocytes could be judged after 3 days of preservation. Moreover, judgement of class 1 antigen and class 2 antigen could be achieved satisfactorily even after 7 days of preservation.

The stimulation indexes (SI) were expressed as a reaction between blood donors A-B divided by a reaction between a blood donor A-A or B-B. When SI is not larger than 2.0. MLC is judged as (-), that is, judged to be compatible. When SI is not less than 5.0, MLC is judged as (+), that is, judged to be not compatible. Fig. 5 shows MLC of the those which were clearly not compatible with each other, and the result that MLC was (+) was obtained at least for 3 days of preservation.

According to the container of the invention for preserving blood for testing, the collecting of blood, the preservation of blood and the component separation of preserved blood can be carried out in a closed circuit, so that the samples can be kept asepsis. In addition to this effect, the container has proper oxygen permeability, so that the blood for testing can be preserved at least for not less than 3 days at room temperature. Furthermore, according to the invention, the measurement of the ratio of surface markers of lymphocytes, the judgement of the compliment and the like are possible even after 3 days after blood is collected. Moreover, since the container according to the invention is constructed using soft plastics, such problems as suffering from damage during transportation can be avoided.

## Claims

1. A container for preserving blood to be supplied to a HLA histocompatible test, in which lymphocytes contained in the blood are kept for at least three days at room temperature, while maintaining the viability of the lymphocytes not less than 90 %, characterized by providing
a main body container (2) composed of a flexible synthetic resin having oxygen permeability of not less than 0.1 ml/24 h·atm per 1 ml of a whole blood, and having a capacity of 20 to 80 ml,
a sampling port portion (8) provided in said main body container (2) at an upper end thereof, having a rubber made button (8a) for piercing a needle, and
an anti-coagulant agent enclosed in advance in said main body container.

2. The container for preserving the blood to be tested as claimed in claim 1, characterized by providing blood collecting means (3) furnished with under mentioned structure in the main body container (2),
a tube (6) having a pricking needle (7) at its end portion for introducing the blood from a donor into the container through the needle,
a connecting member (9) interposed on the way of said blood introducing tube (6), having a closing member for closing the flow path of said tube for introducing the blood when not used and having a structure that the flow path is opened by breaking the closing member when used,
a sampling port portion (12) interposed on the way of said blood introducing tube (6), having a rubber made button for piercing a needle, and
a clamp (10) provided between the sampling port portion (12) of the blood introducing tube (6) and the connecting member (9).

3. The container for preserving the blood to be tested as claimed in claim 2, characterized by providing
a first connecting tube (2) connected to the main body container (2) via a connecting member (5), having branched tubes (16, 17),
a first container (4a) for holding blood components, connected to one branched tube (16) of said first connecting tube (20) and having a sampling port portion (18) on the upper end thereof,
a second container (4b) for holding the blood components, connected to the other branched tube (17) of said first connecting tube (20) via a connecting member (14),
a container (4c) for enclosing a diluting liquid, connected to said second container (4b) via the second connecting tube (18),
a sampling port portion (13) provided in said second container (4b) and a connecting mouth of the second connecting tube (18), and
a connecting member (15) provided in said diluting liquid container (4c) and the connecting mouth of the second connecting tube (18).

4. The container for preserving the blood to be tested as claimed in claim 3, characterized in that said connecting members (5, 9, 14, 15) are integrally formed with a large diametered tubular piece (9c) of synthetic resin and a small diametered piece (9b) for closing the upper end of said large diametered piece (9c), and when the small diametered piece is broken, the upper end of the large diametered piece (9c) is opened, so that the paths of said blood introducing tubes (6, 20, 18) are opened.

## Patentansprüche

1. Behälter zur Aufbewahrung von Blut, das einem HLA-Histokompatibilitäts-Test zuzuführen ist, in welchem in dem Blut enthaltene Lymphozyten für mindestens drei Tage bei Raumtemperatur gehalten werden, wobei die Lebensfähigkeit der Lymphozyten bei nicht weniger als 90 % gehalten wird, gekennzeichnet durch
einen Hauptkörperbehälter (2), der aus einem flexiblen synthetischen Harz mit einer Sauerstoffpermeabilität von nicht weniger als 0,1 ml/24 h · atm pro 1 ml von Vollblut gebildet ist, und eine Kapazität von 20 bis 80 ml hat,
einen Probeentnahmeanschlußbereich (8), der in dem Hauptkörperbehälter (2) an einem oberen Ende desselben vorgesehen ist, mit einem aus Gummi gemachten Knopf (8a) zum Einstechen einer Nadel, und
einem Antigerinnungsmittel, das im vorhinein in den Hauptkörperbehälter eingeschlossen ist.

2. Behälter zur Aufbewahrung des zu testenden Bluts nach Anspruch 1, gekennzeichnet durch eine Blutsammeleinrichtung (3), die mit der nachfolgend genannten Struktur in dem Hauptkörperbehälter (2) vorgesehen ist,
einem Schlauch (6) mit einer Einstechnadel (7) an seinem Endbereich zum Einführen des Bluts von einem Spender in den Behälter durch die Nadel,
einem Verbindungselement (9), das das auf dem Weg des Bluteinführungsschlauchs (6) zwischengeschaltet ist, mit einem Verschlußelement zum Verschließen des Strömungswegs des Schlauchs zum Einführen des Bluts, wenn er nicht verwendet wird, und mit einer Struktur, daß der Strömungsweg durch Brechen des Verschlußelements geöffnet wird, wenn er verwendet wird,
einem Probeentnahmeanschlußbereich (12), der auf dem Weg des Bluteinführungsschlauchs (6) zwischengeschaltet ist, mit einem aus Gummi gemachten Knopf zum Einstechen einer Nadel, und
einer Klemme (10), die zwischen dem Probeentnahmeanschlußbereich (12) des Bluteinführungsschlauchs (6) und dem Verbindungselement (9) vorgesehen ist.

3. Behälter zum Aufbewahren des zu testenden Bluts nach Anspruch 2, gekennzeichnet durch
einen ersten Verbindungsschlauch (2), der mit dem Hauptkörperbehälter (2) verbunden ist über ein Verbindungselement (5) mit verzweigten Schläuchen (16, 17),
einen ersten Behälter (4a) zum Halten von Blutbestandteilen, der mit einem verzweigten Schlauch (16) des ersten Verbindungsschlauchs (20) verbunden ist und einen Probeentnahmeanschlußbereich (18) am oberen Ende desselben aufweist,
einen zweiten Behälter (4b) zum Halten der Blutbestandteile, der mit dem anderen verzweigten Schlauch (17) des ersten Verbindungsschlauchs (20) über ein Verbindungselement (14) verbunden ist,
einen Behälter (4c) zum Einschließen einer Verdünnungsflüssigkeit, der mit dem zweiten Behälter (4b) über den zweiten Verbindungsschlauch (18) verbunden ist,
einen Probeentnahmeanschlußbereich (13), der in dem zweiten Behälter (4b) und einer Anschlußmündung des zweiten Verbindungsschlauchs (18) vorgesehen ist, und
ein Verbindungselement (15), das in dem Verdünnungsflüssigkeitsbehälter (4c) und der Anschlußmündung des zweiten Verbindungsschlauchs (18) vorgesehen ist.

4. Behälter zur Aufbewahrung des zu testenden Bluts nach Anspruch 3, dadurch gekennzeichnet, daß die Verbindungselemente (5, 9, 14, 15) integral ausgebildet sind mit einem einen großen Durchmesser aufweisenden röhrenförmigen Stück (9c) aus synthetischem Harz und einem einen kleinen Durchmesser aufweisenden Stück (9b) zum Verschließen des oberen Endes des den großen Durchmesser aufweisenden Stücks (9c), und wenn das den kleinen Durchmesser aufweisende Stück zerbrochen ist, das obere Ende des den großen Durchmesser aufweisenden Stücks (9c) geöffnet ist, so daß die Wege der Bluteinführungsschläuche (6, 20, 28) geöffnet sind.

## Revendications

1. Récipient pour conserver le sang destiné à des tests d'histocompatibilité de HLA, dans lequel les lymphocytes contenus dans le sang sont maintenus pendant au moins 3 jours à la température ambiante, tout en maintenant la viabilité des lymphocytes à pas moins de 90%, caractérisé en ce qu'il est prévu
un récipient dont le corps principal (2) est composé en une résine synthétique flexible présentant une perméabilité à l'oxygène qui n'est pas inférieure à 0,1 ml/24h/atm par 1 ml de sang entier et dont la capacité est de 20 à 80 ml,
une portion d'orifice d'échantillonnage (8) prévue dans ledit corps principal (2) du récipient à son extrémité supérieure, comportant un bouton (8a) réalisé en caoutchouc et destiné à être percé par une aiguille, et
un agent anti-coagulant introduit à l'avance dans ledit corps principal du récipient.

2. Récipient pour conserver le sang destiné à des tests selon la revendication 1, caractérisé en ce qu'il est prévu
un moyen collecteur de sang (3) équipé de la structure mentionnée ci-dessous dans le corps principal (2) du récipient,
un tube (6) comportant une aiguille perceuse (7) dans sa partie d'extrémité pour introduire le sang provenant d'un donneur dans le récipient par l'intermédiaire de l'aiguille,
un élément de liaison (9) interposé sur le parcours dudit tube d'introduction de sang (6), comportant un élément de fermeture pour fermer le parcours d'écoulement dudit tube destiné à l'introduction du sang quand il n'est pas utilisé, et présentant une structure telle que le parcours d'écoulement est ouvert par rupture de l'élément de fermeture en vue de l'utilisation,
une portion à orifice d'échantillonnage (12) interposée sur le parcours dudit tube d'introduction de sang (6) et comprenant un bouton réalisé en caoutchouc et destiné à être percé par une aiguille, et
une pince (10) prévue entre la portion à orifice d'échantillonnage (12) du tube d'introduction de sang (6) et l'élément de liaison (9).

3. Récipient pour conserver le sang destiné à des tests selon la revendication 2, caractérisé par
un premier tube de liaison (2) relié au corps principal (2) du récipient par l'intermédiaire d'un élément de liaison (5) comportant des tubes de branchement (16, 17),
un premier récipient (4a) pour conserver des composants du sang, relié à un tube de branchement (16) dudit premier tube de liaison (20) et comportant une portion à orifice d'échantillonnage (18) sur son externe supérieure,
un second récipient (4b) pour contenir des composants du sang, relié à l'autre tube de branchement (17) dudit premier tube de liaison (20) par l'intermédiaire d'un élément de liaison (14),
un récipient (4c) pour contenir un liquide de dilution relié audit second récipient (4b) par l'intermédiaire du second tube de liaison (18),
une portion à orifice d'échantillonnage (13) prévue dans ledit second récipient (4b) et une embouchure de liaison du second tube de liaison (18), et
un élément de liaison (15) prévu dans ledit récipient à liquide de dilution (4c) et l'embouchure de liaison du second tube de liaison (18).

4. Récipient pour conserver le sang destiné à des tests selon la revendication 3, caractérisé en ce que lesdits éléments de liaison (5, 9, 14, 15) sont formés d'un seul tenant avec un élément tubulaire de grand diamètre (9c) en résine synthétique et un élément de petit diamètre (9b) pour fermer l'extrémité supérieure dudit élément de grand diamètre (9c), et quand l'élément de petit diamètre est brisé, l'extrémité supérieure de l'élément de grand diamètre (9c) est ouverte de manière que les parcours desdits tube d'introduction de sang (6, 20, 18) soient ouverts.
